(19) Europäisches Patentamt
European Patent Office
Office européen des brevets

(11) Publication number: **0 188 697 A2**

# (12) EUROPEAN PATENT APPLICATION

(21) Application number: **85114923.7**

(22) Date of filing: **25.11.85**

(51) Int. Cl.⁴: **C 07 H 13/06**, A 61 K 31/70

(30) Priority: **26.11.84 JP 249019/84**
**18.05.85 JP 106295/85**

(43) Date of publication of application: **30.07.86**
**Bulletin 86/31**

(84) Designated Contracting States: **CH DE FR GB IT LI NL SE**

(71) Applicant: **Toho Yakuhin Kogyo Kabushiki Kaisha, Yachiyo Building 1-14, Awaji-machi, Higashi-ku Osaka-shi Osaka-fu (JP)**

(72) Inventor: **Hasegawa, Akira, 1735-160, Kano-okurayama, Gifu-shi Gifu-ken (JP)**
Inventor: **Kiso, Makoto, 57-47, Monju Motosu-cho, Motosu-gun Gifu-ken (JP)**
Inventor: **Homma, Yuzuru, 2-34-7, Daizawa, Setagaya-ku Tokyo-to (JP)**
Inventor: **Matsu'ura, Motohiro, Fuji Haitsu 101 6-2-3, shimo'uma, Setagaya-ku Tokyo-to (JP)**
Inventor: **Morihara, Kazuyuki, 5-9-2, Hirose Shimamoto-cho, Mishima-gun Osaka-fu (JP)**

(74) Representative: **Vossius Vossius Tauchner Heunemann Rauh, Siebertstrasse 4 P.O. Box 86 07 67, D-8000 München 86 (DE)**

(54) **2-deoxy-4-0-phosphoryl-2-[(3'-0-tetradecanoyl)-tetradecanoylamino]-3-0-tetradecanoyl-D-glucose and stereoisomers thereof.**

(57) The subject matter of the invention is the racemate as well as the (R)- and (S)-forms of 2-deoxy-4-O-phosphoryl-2-[(3'-O-tetradecanoyl)-tetradecanoylamino]-3-O-tetradecanoyl-D-glucose and their preparation process from known starting compounds. The compounds of the invention exhibit various interesting biological and immunological activities of natural lipid A.

EP 0 188 697 A2

VOSSIUS · VOSSIUS · TAUCHNER · HEUNEMANN · RAUH
PATENTANWÄLTE
0188697

SIEBERTSTRASSE 4 · 8000 MÜNCHEN 86 · PHONE: (089) 47 40 75
CABLE: BENZOLPATENT MÜNCHEN · TELEX 5-29 453 VOPAT D

TOHO YAKUHIN KOGYO KABUSHIKI KAISHA
Our Ref.: U 118 EP
Case: EP/LIPA-1 25.11.1985

# 2-Deoxy-4-O-Phosphoryl-2-[(3'-O-Tetradecanoyl)-Tetradecanoylamino]-3-O-Tetradecanoyl-D-Glucose and Stereoisomers Thereof

This invention relates to 2-deoxy-4-O-phosphoryl-2-[(3'-O-tetradecanoyl)-tetradecanoylamino]-3-O-tetradecanoyl-D-glucose and stereoisomers thereof. These novel compounds have been found by the present inventors in the course of investigating an effective molecular moiety, which is supposed to generate a greater part of the biological and immunological activities which are exhibited by natural lipid A.

Lipo-polysaccharides, hereinafter referred to as "LPS" in this specification, are found in the cell-wall of some kinds of gram-negative bacilli as a main component of endotoxin. They exhibit various kinds of biological and immunological activities such as an anti-tumor activity. Lipid A is a lipoid component of LPS. It is known that the biological and immunological activities of LPS mostly depend on the lipid A component.

0188697

In an attempt to eludicate the chemical structure of lipid A, and to synthesize analogs of lipid A which exhibit as many biological and immunological properties of natural lipid A as possible compounds of the following formula have been described by Galanos and Ludritz et al. in 1977 (cf. Int. Rev. Biochem. 14: 239 (1977) and Naturwissensch.65: 578 (1978))

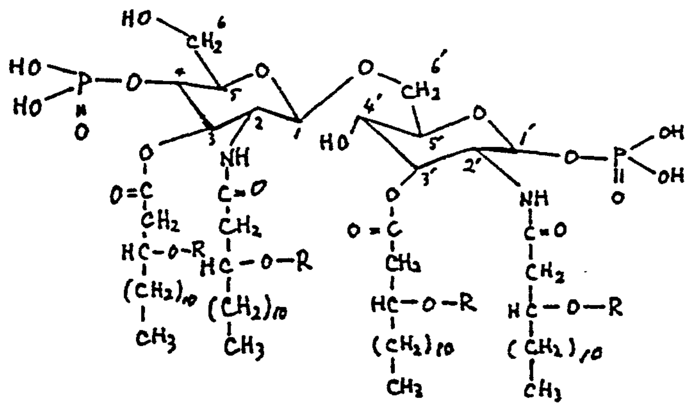

In the above formula, R represents a hydrogen atom or the residue of straight chain aliphatic acid having 12 to 16 carbon atoms, especially myristic acid /$CH_3(CH_2)_{12}COOH$/.

The compounds of the above structure are characterized by two glucosamine groups which are linked together at their 1- and 6'-positions. The amino groups are located at the 2- and 2'-positions. The hydroxy groups are located at the 3- and 3'-positions of the glucosamine groups. 3-Hydroxy-myristic acid residues are attached by an amide or an ester linkage. The phosphoric acid groups are linked to the 1- and 4'-positions, respectively of the glucosamine groups. The compounds thus simultaneously have both hydrophilic and lipophilic substituents on the glucosamine groups.

In the above chemical formula, the left side glucosamine group is called the non-reducing subunit.

On the assumption that it is the non-reducing subunit where the biological and immunological activities of lipid A are mainly concentrated, the inventors have carried out extensive research to synthesize analogs of the non-reducing subunit of lipid A, and have achieved the present invention.

Of the derivatives of the non-reducing subunit of lipid A which have been synthesized by the present inventors, 2-deoxy-4-O-phosphoryl-2-[(3'-O-tetradecanoyl)-tetradecanoylamino]-3-O-tetradecanoyl-D-glucose represented by the formula;

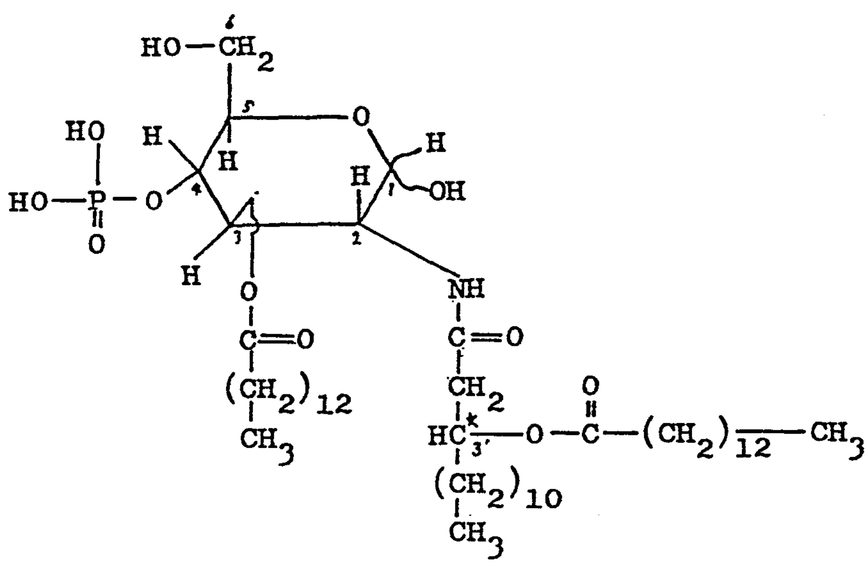

wherein the carbon atom which is marked with an asterisk * is an asymmetric one, and its stereoisomers of a rectus configuration, hereinafter referred to as an (R) type, and a sinister configuration, hereinafter referred to as a (S) type, to possess the most interesting biological and immunological properties.

The following nomenclature is employed in this specification in accordance with a textbook of sugar chemistry:

α-D-glucopyranose;

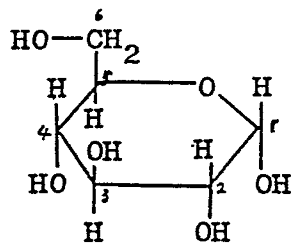

(α)

(RS)-2-deoxy-4-O-phosphoryl-2-[(3'-O-tetradecanoyl)-tetradecanoylamino]-3-O-tetradecanoyl-D-glucose is obtainable from the known benzyl 2-deoxy-2-(3'-hydroxy-tetradecanoylamino)-4,6-O-isopropylidene-ß-D-glucopyranoside, which may be prepared according to the process described in Agric. Biol. Chem.; Vol. 48, pages 251 - 252 (1984). This compound is esterified with myristoyl chloride. Then the obtained product is subjected to a mild hydrolysis to give benzyl 2-deoxy-2-[(3'-O-tetradecanoyl)-tetradecanoylamino]-3-O-tetradecanoyl-ß-D-glucopyranoside and after tritylation of the 6-hydroxy group of the product, quantitative phosphorylation of the 4-hydroxy group with diphenylphospho-chloridate is carried out. Removal of all the protective groups finally gives the objective compound.

The racemate, that is the (RS)-mixture of 2-deoxy-4-O-phosphoryl-2-[(3'-O-tetradecanoyl)-tetradecanoylamino]-3-tetradecanoyl-D-glucose may be optically resolved into the corresponding stereoisomers of the (R)-type and (S)-type.

The invention also relates to the side by side preparation of the (R)-type and the (S)-type of 2-deoxy-4-O-phosphoryl-2-[(3'-O-tetradecanoyl)-tetradecanoylamino]-3-O-tetradecanoyl-D-glucose from the known benzyl 2-acetoamido-2-deoxy-4,6-O-isopropylidene-ß-D-glucopyranoside. This starting compound was disclosed by A. Hasegawa & H. G. Fletcher Jr. in Carbohydrates Research: Vol. 29, pages 209 - 222 (1973).

## EXAMPLES

Example 1: Preparation of (RS)-2-deoxy-4-O-phosphoryl-2-[(3'-O-tetradecanoyl)-tetradecanoylamino]-3-O-tetradecanoyl-D-glucose

[Step a] Preparation of benzyl 2-deoxy-2-(3'-hydroxytetradecanoylamino)-4,6-O-isopropylidene-ß-D-glucopyranoside. [Introduction of a 3'-hydroxytetradecanoyl group into the 2-amino group]

Following the process disclosed by some of the present inventors et al. in Agric. Biol. Chem.: M. Kiso., H. Ishida & A. Hasegawa; Vol. 48, pages 251 - 252 (1984), at first, 0.25 g of 3-hydroxymyristic acid (3-hydroxytetradecanoic acid) was dissolved in anhydrous 1,4-dioxane (8 ml), to which n-hydroxysuccinimide (0.16 g) and dicyclohexylcarbodiimide (0.3 g) were added. The reaction mixture was well stirred at room temperature to activate said 3-hydroxymyristic acid. To the thus activated reaction mixture, benzyl 2-deoxy-2-amino-4,6-isopropyliden-ß-D-glucopyranoside (0.43 g) was added and the mixture was stirred at room temperature for a night. Then, the by-produced dicyclohexyl-urea was removed by filtration from the reaction mixture and the filtrate was concentrated in vacuo to give a

syrupy material, which was subjected to column chromatography (Waco gel C-200, solvent for elution; chloroform: methanol = 100:1 v/v) to give the title compound (0.56 g, 80 %).

[Step b]; Preparation of benzyl 2-deoxy-2-[( 3'-O-tetradecanoyl)-tetradecanoylamino]-3-O-tetradecanoyl-β-D-glucopyranoside [ Introduction of a tetradecanoyl group into the 3-hydroxy and 3'-hydroxy groups and removal of the 4,6-O-isopropylidene group]

The product (0.2 g) of the preceding Step a. was treated with myristoyl chloride in the same manner as in the preceding Step a. and the produced syrupy product was subjected to column chromatography to obtain benzyl 2-deoxy-4,6-O-isopropylidene-2-[(3'-O-tetradecanoyl)-tetradecanoylamino]-3-O-tetradecanoyl-β-D-glucopyranoside.

$[\alpha]_D$ -30° (C 0.8, $CHCl_3$).

IR$\nu_{max}^{film}$ $cm^{-1}$: 3340 (NH), 1740 (ester), 1660, 1540 (amide), 860 ($Me_2C$), 730 - 700 (ph).

NMR data (90MHz, $CDCl_3$) $\delta$: 0.75-0.95 (9H, Me), 1.1-- 1.7, 2.05 - 2.45 (70H, methylene), 1.32, 1.95 (6H, 2s, $Me_2C$), 7.23 (5H, ph).

The product was subjected to hydrolysis to remove the isopropylidene group. The title compound was obtained in a quantitative yield.

$[\alpha]_D$ -20° (C 1.7, $CHCl_3$).

IR$\nu_{max}^{film}$ $cm^{-1}$: 3500-3000 (OH, NH), 1710-1740 (ester), 1640, 1550 (amide), 75o-680 (ph).

[Step c]; Preparation of benzyl 2-deoxy-2-[( 3'-O-tetradecanoyl)-tetradecanoylamino]-3-O-tetradecanoyl-6-O-trityl-β-D-glucopyranoside [ Tritylation of the 6-hydroxy group]

The product ( 0.2 g) obtained in the preceding Step b. was dissolved in anhydrous pyridine (4 ml), to which trityl chloride (0.13 g) was added at 90°C. The mixture was stirred for several hours. After the reaction, an excess amount of methanol was added and the mixture was concentrated in vacuo. The produced syrup was extracted with chloroform and the extracts were subjected to column chromatography, which gave the title product (79 %).

$[\alpha]_D$ -22.5°

IR$\nu^{film}_{max}$ $cm^{-1}$: 3600-3200 (OH, NH), 1740 (ester), 1650, 1560 (amide), 780-700 (ph).

[Step d]; Preparation of benzyl 2-deoxy-4-O-diphenylphosphoryl-2-[( 3'-O-tetradecanoyl)-tetradecanoylamino]-3-O-tetradecanoyl-β-D-glucopyranoside [Diphenylphosphorylation of the 4-hydroxy group and de-tritylation]

The product (0.27 g) of the preceding Step c.was dissolved into a mixed solvent of anhydrous dichloromethane (2 ml) and anhydrous pyridine (1 ml), to which dimethylaminopyridine (0.06 g) was added and the mixture

was cooled to 0°C under stirring. To the thus produced reaction mixture, a solution of diphenylphosphochloridate (0.2 g) dissolved in anhydrous dichloromethane (1 ml) was dropped and was stirred for a night at room temperature. An excess quantity of methanol was added to the reaction mixture which was then concentrated in vacuo. The residue was extracted with chloroform and the obtained phosphorylated compound was dissolved in a mixed solvent of chloroform and methanol, to which acetic acid and water were added and the mixture was stirred at 45°C. The reactant was concentrated in vacuo and the thus produced material subjected to column chromatography (Waco gel C-200, solvent for elution; chloroform:methanol =200:1 v/v). The title compound was obtained in a yield of 95 %.

$[\alpha]_D$ -28° (C 0.8, chloroform).

IR$\nu_{max}^{film}$ $cm^{-1}$: 3480 (OH), 3280 (NH), 1740 (ester), 1650, 1560 (amide), 960 ( P-O-ph), 800-680 (ph).

NMR data (90MHz, $CDCl_3$) : 0.75-1.0 (9H, Me), 1.0-2.45 (70H, methylene), 7.0-7.45 (15H, ph).

Elemental analysis(%): For $C_{67}H_{106}NO_{12}P$

Calcd.: C, 70.06; H, 9.30;

Found : C, 70.35; H, 9.07

[Step e]; Preparation of the final objective compound [De-benzylation of the 1-hydroxy group and removal of the diphenyl group from the 4-O-diphenylphosphoryl group]

The product of the preceding Step d. was dissolved in a mixed solvent of ethanol and methanol, to which palladium on carbon, which had preliminarily been subjected to reduction, was added. The mixture was subjected to hydrogenation according to conventional means. The 1-O-benzyl group was removed quantitatively. The remaining catalyst was removed by filtration from the reaction mixture and the filtrate was concentrated in vacuo. The thus obtained residue was hydrogenated with platinum oxide, which had preliminarily been subjected to reduction, for removing the diphenyl group from the 4-O-diphenyl-phosphoryl group. The reaction mixture was filtered with Cellites and the filtrate was concentrated in vacuo. The product was lyophilized from a suspension in 1,4-dioxane, to obtain the final objective compound quantitatively as a colorless powder.

Melting point: 159 - 162 °C.(Decomposed)

$[\alpha]_D$ + 13° (C 0.3, Dimethylformamide containing 0.01 % of triethylamine).

Example 2: Side-by-side preparation of (R)- and (S)-type of 2-deoxy-4-O-phosphoryl-2-[(3'-O-tetradecanoyl)-tetradecanoylamino]-3-O-tetradecanoyl-D-glucose.

[Step a] Preparation of benzyl 2-amino-2-deoxy-4,6-O-isopropylidene-β-D-glucopyranoside [De-acetylation of the known starting compound].

10 g of 2-acetoamido-2-deoxy-4,6-O-isopropylidene-ß-D-glucopyranoside [Carbohydrates Research; Vol. 29, pages 209 - 222 (1973)] was added to a mixture of water (150 ml) and barium hydroxide (18 g), and the mixture was refluxed for a night under stirring. The reaction mixture was warmed to room temperature and extracted with chloroform. The chloroform layer was washed with water and dried over anhydrous sodium sulfate and then concentrated. The thus obtained syrupy material was crystallized from a mixed solvent of ether and hexane, to give 8.5 g of the title objective compound in a yield of 97 %.

Melting point: 127.5 - 130 °C.

$[\alpha]_D$ -95.45° (C 1.03, chloroform).

IR $\nu_{max}^{film}$ $cm^{-1}$: 3380, 3330 ($NH_2$), 3300 - 2600 (OH), 855 ($Me_2C$), 740, 710, 690 (ph).

NMR data (60MHz, $CDCl_3$) $\delta$ : 7.3 (5H, s, ph), 4.90, 4.53 (2H, dd, $J_{gem}$, 12Hz, -$\underline{CH_2}$ph), 4.33 (1H, d, $J_{1,2}$, 8Hz, H-1), 1.43, 1.50 (6H, 2S, $Me_2C$).

Elemental analysis for $C_{16}H_{23}NO_5$ = 309.35

Calcd.(%): C, 62.12; H, 7.49; N, 4.53

Found (%): C, 62.08; H, 7.48; N, 4.42

[Step b] Side-by-side preparation of (R)- and (S)-type of benzyl 2-deoxy-(3'-hydroxytetradecanoylamino)-4,6-O-isopropylidene-$\beta$-D-glucopyranoside [hydroxytetradecanoylation of the 2-amino group]

The compound obtained in the preceding Step a. (3.0 g, 0.0094 mol) was dissolved in dioxane (45 ml).

Hydroxytetradecanoic acid (6.87 g, 2.9 mol equivalent) and dicyclohexylcarbodiimide (19.2 g, 4.8 mol equivalent to the quantity of the acid) were added divided in several portions under stirring. Completion of the reaction was confirmed by thin layer chromatography (chloroform: methanol = 10:1 v/v). The reaction mixture was filtered and the precipitate was washed with dioxane. The filtrate and the washing solvent were combined and concentrated. The syrupy material obtained was subjected to middle-speed column chromatography (Waco gel C-300, solvent for elution; chloroform:methanol = 100:1 v/v). The (R)-type of the title compound (22 g) and the (S)-type of the title compound (1.9 g) and a mixture of (R)- and (S)-type (1.0 g) were obtained. The mixture of (R)- and (S)-type product was separated into the stereoisomers by repeating the column chromatography.

The structure of the stereoisomers was identified according to the method described by M. Kiso et al. in Carbohydrates Research: Vol. 88, C-5 - C-9 (1981), by converting them to the derivative benzyl 3,4,6-tri-O-acetyl-2-(3'-O-acetyltetradecanoylamino)-2-deoxy-ß-D-glucopyranoside.

(R)-type compound:

Melting point: 99 - 102°C.

$[\alpha]_D$ -69.87 (C 0.664, chloroform).

IR $\nu_{max}^{film}$ $cm^{-1}$ : 3200 - 3700 (NH, OH), 1730 (ester), 1560, 1650 (amide), 860 ($Me_2C$), 700 (ph).

NMR data (90 MHz, $CDCl_3$) : 0.75 - 1.0 (3H, Me), 1.1 - 1.6 (20H, methylene), 1.37, 1.48 (6H, 2S, $Me_2C$), 2.15 - 2.35 (2H, $-COCH_2-$), 4.53, 4.87 (2H, 2d, $-CH_2$-ph), 6.79 (1H, d, -NH-), 7.2 - 7.4 (5H, ph), 4.90 (1H, d, H-1).

(S)-type compound

Melting point: 131 - 131.5°C.

$[\alpha]_D$ -47.45° (C 0.569, chloroform).

IR $\nu_{max}^{film}$ $cm^{-1}$: 3200 - 3700 (NH, OH), 1710 (ester), 1660, 1550 (amide), 860 ($Me_2C$), 700 (ph).

NMR data (90MHz, $CDCl_3$) : 0.75 - 1.0 (3H, Me), 1.1 - 1.6 (20H, methylene), 1.40, 1.53 (6H, 2S, $Me_2C$), 2.20 - 2.45 (2H, $-COCH_2-$), 4.53, 4.82 (2H, 2d, $-\underline{CH}_2$-ph), 4.55 (1H, d, H-1), 7.10 (1H, d, -NH-), 7.2 - 7.4 (5H, ph).

[Step c] Side-by-side preparation of (R)- and (S)-type of benzyl 2-deoxy-4,6-O-isopropylidene-3-O-tetradecanoyl-2-[(3'-O-tetradecanoyl)-tetradecanoylamino]-ß-D-glucopyranoside [Tetradecanoylation of the 3'-hydroxy group]

0.83 grams (0.0015 mol) of the (R)-type compound obtained in the preceding step b. or 0.86 grams (0.0016 mol) of the (S)-type compound obtained in the preceding step b., were separately dissolved in a mixture of dichloromethane (5ml; 6 ml) and pyridine (2.5 ml; 3 ml). [The first value mentioned in the parenthesis refers to the preparation of the (R)-type compound, the second value to the preparation of the (S)-type compound; this also applies to the following description.]

To the mixed solution, dimethylaminopyridine (0.195 g, 1.0 mol equivalent; 0.20 g, 1.0 mol equivalent) and then, myristic chloride (0.95 g, 2.4 mol equivalent; 0.98 g, 2.4 mol equivalent) was added at 0°C under stirring, which was continued for a night at room temperature. Completion of the reaction was confirmed by thin layer chromatography (chloroform:methanol = 100:1 v/v). Methanol was added to the reaction mixture which was then concentrated in vacuo. The thus obtained syrupy material was extracted with chloroform and the chloroform layer was washed with 2N hydrochloric acid, and water and dried over anhydrous sodium sulfate and concentrated in vacuo. The obtained syrupy material was subjected to a column chromatography (Waco gel C-300, solvent for elution; chloroform). The syrupy material obtained, was crystallized from a mixture of ether and hexane. 1.15 g (78 %) of the title (R)-type compound and 1.25 g (85 %) of the title (S)-type compound were obtained.

(R)-type compound

Melting point: 86 - 87 °C.

$[\alpha]_D$ -32.12° (C 1.077, chloroform).

IR $\nu^{film}_{max}$ $cm^{-1}$: 3410 (NH), 1740 (ester), 1680, 1520 (amide), 860($Me_2C$).

NMR data (270MHz, $CDCl_3$) $\delta$: 0.75 - 1.0 (9H, Me), 1.1 - 1.6 (64H, methylene), 1.35, 1.47 (6H, 2s, Me C), 2.1-2.5 (6H, $-COCH_2-$), 3.35 (1H, m, $J_{4,5}$ = $J_{5,6a}$ = 10Hz, $J_{5,6b}$ = 5.5Hz, H-5), 3.75 (1H, t, $J_{3,4}$ = $J_{4,5}$ = 10Hz, H-4), 3.82 (1H, t, $J_{5,6a}$ = 10Hz, $J_{gem}$ = 10.6Hz, H-6a), 3.97 (1H, dd,

$J_{gem}$ = 10.6Hz, $J_{5,6b}$ = 5,5Hz, H-6b), 4.11 (1H, m, $J_{1,2}$ = $J_{2,3}$ = $J_{2,NH}$ = 9 - 10Hz, H-2), 4.53 (1H, d, $J_{1,2}$ = 9Hz, H-1), 4.57, 4.86 (2H, 2d, $J_{gem}$ = 12Hz, $-CH_2$-ph), 5.03 (1H, t, H-3), 5.91 (1H, d, $J_{NH,2}$ = 9Hz, -NH-), 7.2 - 7.4 (5H, ph).

(S)-type compound

Melting point: 80 - 81°C.

$[\alpha]_D$ -34.59° (C 0.847, chloroform).

IR $\nu_{max}^{Nujol}$ $cm^{-1}$: 3380 (NH), 1740, 1720 (ester), 1670, 1520 (amide), 860 ($Me_2C$).

NMR data (270MHz, $CDCl_3$) $\delta$ : 0.75 - 1.0 (9H, Me), 1.1 - 1.6 (64H, methylene), 1.35, 1.46 (6H, 2s, $Me_2C$), 2.1-2.5 (6H, $-COCH_2-$), 3.37 (1H, m, $J_{4,5}$ = $J_{5,6}$ = 10, $J_{5,6b}$ = 5.5Hz, H-5), 3.74 (1H, t, $J_{3,4} \simeq J_{4,5}$ = 10Hz, H-4), 3.81 (1H, t, $J_{5,6a}$ = $J_{gem}$ = 10Hz, H-6a), 3.96 (1H, dd, H-6b), 4.14 (1H, m, $J_{1,2}$ = 8.1, $J_{2,3}$ = $J_{2,NH'}$ = 10Hz, H-2), 4.54 (1H, d, H-1), 4.56, 4.86 (2H, 2d, $J_{gem}$ = 12Hz, $-CH_2$-ph), 5.06 (1H, t, $J_{2,3}$ = $J_{3,4}$ = 10Hz, H-3), 5.98 (1H, d, $J_{NH,2}$ = 10Hz, -NH-), 7.1 - 7.3 (5H, ph).

[Step d] Side-by-side preparation of (R)- and (S)-type of benzyl 2-deoxy-3-O-tetradecanoyl-2-[(3'-O-tetradecanoyl)-tetradecanoylamino]-$\beta$-D-glucopyranoside [Removal of the isopropylidene group from the 4,6-hydroxy group].

0.98 g (0.0010 mol) of the (R)-type compound or 0.75 g (0.0008 mol) of the (S)-type compound obtained in the preceding Step c. were separately dissolved in a mixture of 90 % aqueous acetic acid containing small quantities of chloroform and methanol. The solution was allowed to stand for a while at 45°C. Completion of the reaction was confirmed by thin layer chromatography (chloroform: methanol = 50:1 v/v). The reaction mixture was concentrated in vacuo. The thus obtained syrupy material was subjected to column chromatography (Waco gel C-300, solvent for elution; chloroform:methanol 400:1 v/v) to obtain a syrupy material, which was crystallized from a mixture of ether and hexane to give the title compounds (0.905 g, 96 %, crystals; 0.662 g, 92 %, amorphous solid).

<u>(R)-type compound</u>

Melting point: 134 - 135°C.

$[\alpha]_D$ -21.39° (C 0.729, chloroform).

IR $\nu_{max}^{film}$ $cm^{-1}$: 3200 - 3600 (NH, OH), 1740 (ester), 1660, 1560 (amide).

NMR data (270MHz, $CDCl_3$) $\delta$ : 0.75 - 1.0 (9H, Me), 1.1 - 1.7 (64H, methylene), 2.1 - 1.6 (6H, $-COCH_2-$), 3.4 - 3.5 (1H, m, H-5), 4.62, 4.84 (2H, 2d, $J_{gem}$ = 12Hz, $-CH_2$-ph), 4.62 (1H, d, $J_{1,2}$ = 8Hz, H-1), 5.04 (1H, t, $J_{3,2} = J_{3,4}$ = 9Hz, H-3), 6.07 (1H, d, $J_{NH,2}$ = 9Hz, -NH-), 7.1 - 7.4 (5H, ph).

<u>(S)-type compound</u>

$[\alpha]_D$ -23.05° (C 0.746, chloroform).

IR $\nu_{max}^{film}$ $cm^{-1}$: 3200 - 3600 (NH, OH), 1740 (ester),

1660, 1570 (amide).

NMR data (270MHz, $CDCl_3$) δ: 0.75 - 1.0 (9H, Me), 1.1 - 1.7 (64H, methylene), 2.1 - 2.5 (6H, $-COCH_2-$), 3.4 - 3.5 (1H, m, H-5), 4.60, 4.85 (2H, 2d, $J_{gem}$ = 12Hz, $-CH_2-$ph), 5.07 (1H, t, $J_{3,2} = J_{3,4}$ = 10Hz, H-3), 6.18 ( 1H, d, $J_{NH,2}$ - 9Hz, -NH-), 7.1 - 7.4 (5H, ph).

[Step e] Side-by-side preparation of (R)- and (S)-type of benzyl 2-deoxy-3-O-tetradecanoyl-[(3'-O-tetradecanoyl)-tetradecanoylamino]-6-O-trityl-β-D-glucopyranoside [Tritylation of the 6-hydroxy group].

0.915 g (0.0010 mol) of the (R) type compound or 0.661 g (0.0007 mol) of the (S)-type compound obtained in the preceding Step d. were separately dissolved in pyridine (8 ml; 5 ml). The solution was refluxed at 95°C under stirring. Trityl chloride (0.43 g, 1.5 mol equivalent; 0.31 g, 1.5 mol equivalent) was added. Completion of the reaction was confirmed by thin layer chromatography (chloroform:methanol = 20:1 v/v). Methanol was added and the solution was concentrated in vacuo, to obtain a syrupy material, which was extracted with chloroform. The chloroform layer was washed successively with 2N hydrochloric acid, 10 % sodium carbonate and water, dried over anhydrous sodium sulfate and concentrated in vacuo. The obtained syrupy material was subjected to column chromatography (Waco gel C-200, solvent for elution; chloroform), to give a syrupy material (0.99 g, 86 %; 0.61 g, 73 %).

<u>(R)-type compound</u>

$[\alpha]_D$ -25.52° (C 0.721, chloroform).

IR $\nu_{max}^{film}$ $cm^{-1}$: 3200 - 3600 (NH, OH), 1740 (ester), 1650, 1560 (amide), 650 - 800 (ph).

NMR data (90Hz, $CDCl_3$) $\delta$ : 0.8 - 1.0 (9H, Me), 1.1 - 1.7 (64H, methylene), 2.1 - 2.5 (6H, $-COCH_2-$), 4.64, 4.92 (2H, 2d, $-CH_2$-ph), 4.57 (1H, d, H-1), 4.99 (1H, t, H-3), 5.95 (1H, d, -NH-), 7.1 - 7.6 (20H, ph).

<u>(S)-type compound</u>

$[\alpha]_D$ -25.02° (C 1.191, chloroform).

IR $\nu_{max}^{film}$ $cm^{-1}$: 3200 - 3600 (NH, OH), 1740 (ester), 1650, 1560 (amide), 650 - 800 (ph).

NMR data (90Hz, $CDCl_3$) $\delta$ : 0.8 - 1.0 (9H, Me), 1.1 -1.7 (64H, methylene), 2.1 - 2.5 (6H, $-COCH_2-$), 4.63, 4.91 (2H, 2d, $-CH_2$-ph), 4.56 (1H, d, H-1), 4.90 - 5.20 (1H, H-3), 6.02 (1H, d, -NH-), 7.1 - 7.6 (20H, ph).

[Step f] Side-by-side preparation of (R)- and (S)-type of benzyl 2-deoxy-4-O-diphenylphosphoryl-3-O-tetradecanoyl-2-[(3'-O-tetradecanoyl)-tetradecanoylamino]-6-O-trityl-β-D-glucopyranoside [Diphenylphosphorylation of the 4-hydroxy group]

0.70 g (0.0006 mol) of the (R)-type compound or 0.60 g (0.0005 mol) of the (S)-type compound obtained in the preceding Step e. were separately dissolved in a mixture of dichloromethane (1 ml; 0.6 ml) and pyridine (0.5 ml; 0.3 ml). To the solution dimethylaminopyridine (0.04 g, 0.5 mol equivalent; 0.03 g, 0.5 mol equivalent) was added. To the mixture, diphenylphospho-chloridate (0.43 g, 3 mol equivalent; 0.37 g, 3 mol equivalent) was added. The mixture was stirred for a night at room temperature. Then, methanol was added. The reaction mixture was concentrated in vacuo and the obtained syrupy material was extracted with chloroform. The chloroform layer was washed successively with 2N hydrochloric acid, 10 % sodium carbonate and water, dried over anhydrous sodium sulfate and concentrated in vacuo. The syrupy material obtained was subjected to column chromatography (Waco gel C-200, solvent for elution; chloroform). The title compounds were obtained separately (0.59 g, 70.01 %; 0.55 g, 76 %).

<u>(R)-type compound</u>

$[\alpha]_D$ - 8.81° (C 1.066, chloroform).

<u>(S)-type compound</u>

$[\alpha]_D$ -11.06° (C 1.365, chloroform)

IR$\nu^{film}_{max}$cm$^{-1}$: ( common to both stereisomers) 3300 (NH), 1740 (ester), 1650, 1560 (amide), 960 (P-O-ph), 700 (ph).

[Step g] Side-by-side preparation of (R)- and (S)-type of benzyl 2-deoxy-4-O-diphenylphosphoryl-3-O-tetradecanoyl-[(3'-O-tetradecanoyl)-tetradecanoylamino]-β-D-glucopyranoside [De-tritylation of the 6-hydroxy group]

The syrupy stereoisomers respectively obtained in the preceding Step f. were separately dissolved in 95 % aqueous acetic acid (4 ml; 3 ml) and were stirred at 50°C. Completion of the reaction was confirmed by thin layer chromatography (chloroform:methanol = 25:1 v/v). The reaction mixture was concentrated in vacuo and the thus obtained syrupy material was subjected to column chromatography (solvent for elution; chloroform:methanol = 400:1 v/v) to give the title stereoisomers in a syrupy form (0.244 g, 35 % in two steps; 0.313 g, 53 % in two steps).

<u>(R)-type compound</u>

$[\alpha]_D$ -30.51° (C 1.396, chloroform).

IR$\nu_{max}^{film}$ $cm^{-1}$: 3200 - 3600 (NH, OH), 1740 (ester), 1660, 1550 (amide), 960 (P-O-ph), 650 - 800 (ph).

NMR data (270MHz, $CDCl_3$) $\delta$ : 0.8 - 1.0 (9H, Me), 1.1 - 1.7 (64H, methylene), 1.95 - 2.45 (6H, $-COCH_2-$), 3.92 (1H, m, $J_{4,5} = J_{4,3} = J_{4,p}$ = 9Hz, H-4), 4.59, 4.86 (2H, 2d, $J_{gem}$ = 12Hz, $-CH_2$-ph), 4.74 (1H, d, H-1), 4.99 (1H, m, $-CH_2-CH-CH_2-$), 5.45 (1H, near t, H-3), 5.89 (1H, d, $J_{NH,2}$ = 9Hz, -NH-), 7.1 - 7.4 (15H, ph).

<u>(S)-type compound</u>

$[\alpha]_D$ $-29.51^{\circ}$ (C 1.433, chloroform).

IR $\nu_{max}^{film}$ $cm^{-1}$: 3200 - 3600 (NH, OH), 1730 (ester), 1640, 1560 (amide), 960 (P-O-ph), 650 - 800 (ph).

NMR data (90MHz, $CDCl_3$) $\delta$: 0.8 - 1.0 (9H, Me), 1.1 - 1.7 (64H, methylene), 2.0 - 2.5 (6H, $-COCH_2-$), 4.60, 4.88 (2H, 2d, $-CH_2$-ph), 5.51 (1H, near t, H-3), 6.13 (1H, d, -NH-), 7.05 - 7.40 (15H, ph).

[Step h]; Side-by-side preparation of (R)- and (S)-type of 2-deoxy-4-O-diphenylphosphoryl-3-O-tetradecanoyl-2-[(3'-O-tetradecanoyl)-tetradecanoylamino]-D-glucopyranose [De-benzylation of the 1-hydroxy group]

0.24 g (0.00020 mol) of the (R)-type compound or 0.30 g (0.00026 mol) of the (S)-type compound obtained in the preceding step g. were separately dissolved in methanol (20 ml; 20 ml), to which palladium on carbon (0.2 g; 0.3 g), which had been preliminarily subjected to reduction, was added and hydrogenation was conducted. Completion of the reaction was confirmed by thin layer chromatography (chloroform:methanol = 20:1 v/v). The reaction mixture was filtered, the residue was washed with methanol and the filtrate and the methanol used for washing were combined and concentrated in vacuo. The thus obtained syrupy material was subjected to column chromatography (Waco gel C-200, solvent for elution; chloroform: methanol = 200:1 v/v) to give the title stereoisomers (0.168 g, 76 %; 0.257 g, 93 %).

(R)-type compound

$[\alpha]_D$ +3.22° (C 1.240, chloroform).

IR $\nu_{max}^{film}$ $cm^{-1}$: 3200 - 3600 (NH, OH), 1740 (ester), 1650, 1570 (amide), 960 (P-O-ph), 650 - 680 (ph).

NMR data (270MHz, $CDCl_3$) $\delta$: 0.8 - 1.0 (9H, Me), 1.1 - 1.7 (64H, methylene), 2.0 - 2.5 (6H, $-COCH_2-$), 4.75 (1H, m, $J_{4,3}=J_{4,5}=J_{4,p}$= 10Hz, H-4), 5.09 (1H, m, $-CH_2-\underline{C}H-CH_2-$), 5.25 (1H, t, $J_{1,2}$ = $J_{1,OH}$ = 3Hz, H-1), 5.48 (1H, near t, H-3), 6.10 (1H, d, $J_{NH,2}$ = 9Hz, -NH-), 7.1 - 7.5 (10H, ph).

(S)-type compound

$[\alpha]_D$ + 2.30° (C 0.738, chloroform).

IR $\nu_{max}^{film}$ $cm^{-1}$: 3200 - 3600 (NH, OH), 1740 (ester), 1660, 1550 (amide), 960 (P-O-ph), 650 - 800 (ph).

NMR data (270MHz, $CDCl_3$) $\delta$ : 0.8 - 1.0 (9H, Me), 1.1 - 1.7 (64H, methylene), 2.0 - 2.5 (6H, $-COCH_2-$), 4.75 (1H, m, $J_{4,3}$ = $J_{4,5}$ = $J_{4,p}$ = 10Hz, H-4), 5.10 (1H, m, $-CH_2-\underline{C}H-CH_2-$), 5.22 (1H, t, $J_{1,2}$ = $J_{1,OH}$ = 4Hz, H-1), 5.49 (1H, near t, H-3), 6.16 (1H, d, $J_{2,NH}$ = 9Hz, -NH-), 7.1 - 7.5 (11H, ph).

[Step i] Side-by-side preparation of (R)- and (S)-type of the final objective compounds [De-diphenylation of the 4-phosphoryl group]

0.135 g (0.00013 mol) of the (R)-type compound or 0.170 g (0.00016 mol) of the (S)-type compound obtained in the preceding step h. were separately dissolved in methanol (20 ml, 20 ml), to which platinum oxide, which had been preliminarily subjected to reduction, was added and hydrogenation was conducted. Completion of the reaction was confirmed by thin layer chromatography (chloroform: methanol = 10:1 v/v). The reaction mixture was filtered. The residue was washed with methanol and the filtrate and the methanol used for washing were combined and they were concentrated in vacuo and then lyophilized from dioxane to obtain the final objective compounds respectively (0.110 g, 95 %; 0.140 g, 96 %).

<u>(R)-type compound</u>

Melting point: 142 - 143 °C.

IR $\nu_{max}^{KBr} cm^{-1}$: 3200 - 3600 (NH, OH), 1740 (ester), 1640, 1560 (amide).

<u>(S)-type compound</u>

Melting point: 130 - 131 °C.

$[\alpha]_D$ +11.83° (C 0.794, chloroform).

IR $\nu_{max}^{KBr} cm^{-1}$: 3200 - 3600 (NH, OH), 1730 (ester), 1650, 1550 (amide),

The compounds of this invention, the racemate as well as the stereoisomers having (R) and (S) configuration exhibit various interesting biological and immunological activities. For example, they proclot the enzyme of horseshoe crab. They induce interferon- and tumor-necrosis factors. Furthermore, they act as mitogens for polyclonal B cells and as adjuvant, vgl. Y. Kumazawa et al. in Euro I. Immunol. 15 (1985), S. 199-201.

## Patent Claims

1. 2-Deoxy-4-O-phosphoryl-3-O-tetradecanoyl-2-/(3'-O-tetradecanoyl)-tetradecanoylamino/-D-glucose.

2. 2-Deoxy-4-O-phosphoryl-3-O-tetradecanoyl-2-/(3'-O-tetradecanoyl)-tetradecanoylamino/-D-glucose in the form of the racemate.

3. 2-Deoxy-4-O-phosphoryl-3-O-tetradecanoyl-2-/(3'-O-tetradecanoyl)-tetradecanoylamino/-D-glucose in the form of the (R)-isomer.

4. 2-Deoxy-4-O-phosphoryl-3-O-tetradecanoyl-2-/(3'-O-tetradecanoyl)-tetradecanoylamino/-D-glucose in the form of the (S)-isomer.